# EUROPÄISCHE PATENTANMELDUNG

(11) **EP 0 639 560 A1**
(43) Veröffentlichungstag der Anmeldung: **22.02.1995**
(21) Anmeldenummer: 94112178.2
(22) Anmeldetag: 04.08.1994
(51) Int. Cl.: C07C 271/22, C07C 317/22, C07D 207/44, C07D 209/48, C07F 7/10

(54) **Asymmetrisch substituierte Diaminodicarbonsäurederivate und ein Verfahren zu deren Herstellung**

(30) Priorität: 20.08.1993 AT 1683/93
(71) Anmelder: Hafslund Nycomed Pharma AG, A-4020 Linz (AT)
(72) Erfinder: Hiebl, Johann, Dr., A-4030 Linz (AT); Rovenszky, Franz, Dipl.-Ing. Dr., A-4020 Linz (AT)

(57) **Zusammenfassung**

Die Erfindung betrifft neue asymmetrisch substituierte Diaminodicarbonsäurederivate der Formel 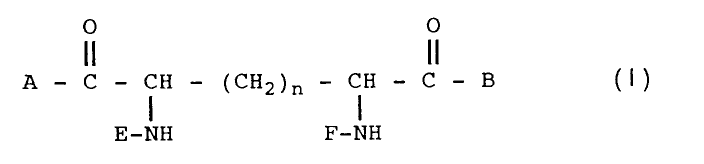
und ein Verfahren zu deren Herstellung durch gemischte Kolbe-Synthese.

## Beschreibung

Die Erfindung betrifft neue asymmetrisch substituierte Diaminodicarbonsäurederivate und ein Verfahren zu deren Herstellung.

Asymmetrisch substituierte Diaminodicarbonsäurederivate sind wertvolle Zwischenprodukte für die Synthese von Peptiden.

In J. Org. Chem. 1980, 45, 3078-3080 sind asymmetrisch substituierte Diaminosuberinsäurederivate beschrieben, die durch gemischte Kolbe-Synthese hergestellt wurden. Eine Abtrennung der symmetrischen Nebenprodukte gelang dort nicht. Ferner ist aus Tetrahedron Lett. 30, 1982, 33, 4727-4730, die Herstellung asymmetrisch substituierter Diaminopimelinsäurederivate durch eine komplizierte 9-stufige enantioselektive Synthese bekannt.

Unerwarteterweise konnten neue asymmetrisch substituierte Diaminodicarbonsäurederivate gefunden werden, die wertvolle Zwischenprodukte für die Synthese von Peptiden und unnatürliche Aminosäuren enthaltende Verbindungen darstellen.

Gegenstand der Erfindung sind daher asymmetrisch substituierte Diaminodicarbonsäurederivate der Formel 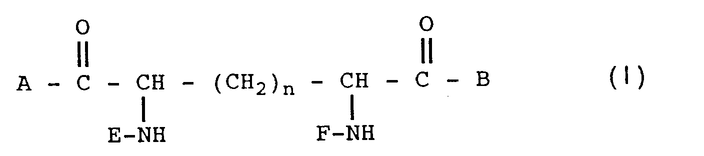
in der
A und B unabhängig voneinander jeweils einen Rest -OR, wobei R einen gegebenenfalls ein- oder mehrfach halogenierten geradkettigen, verzweigten oder zyklischen Alkylrest mit 1-10 C-Atomen, Phenyl oder den Rest -CH₂-X, wobei X die Reste 9-Fluorenyl-, Phenyl-, -OCH₃, -CH₂SO₂CH₃, -CH₂SO₂C₆H₅, -CCl₃, -CH₂-Y, mit Y Halogen, -p-Tosyl, einem gegebenenfalls ein- oder mehrfach durch Halogen, -NO₂ oder Alkoxy substituierten Phenyl- oder Phenacylrest, Diphenylmethyl, Triphenylmethyl, 2-Pyridyl-, oder einen Rest SiR₁R₂R₃, wobei die Reste R₁R₂R₃ jeweils unabhängig voneinander einen geradkettigen oder verzweigten Alkylrest mit 1-4 C-Atomen oder Phenyl bedeuten können, bedeutet,
E,F jeweils einen gegebenenfalls halogenierten geradkettigen, verzweigten oder zyklischen Alkylrest mit 1-10 C-Atomen oder einen Rest 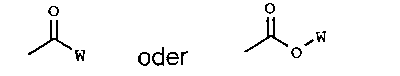
bedeuten, wobei W 9-Fluorenylmethyl- oder einen gegebenenfalls ein- oder mehrfach oder gemischt durch Halogen, -NO₂, Alkoxy oder -CN substituierten Benzylrest bedeuten kann, oder die Substituenten E bzw. F, bei Wegfall des Wasserstoffatoms, gemeinsam mit dem Stickstoffatom eines der folgenden Ringsysteme bilden 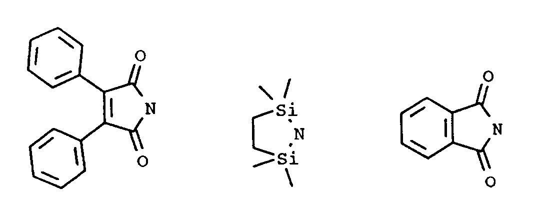
wobei, wenn die Reste A und B unterschiedlich sind, E und F unterschiedlich oder gleich sein können, hingegen wenn die Reste A und B gleich sind, E und F unterschiedlich sein müssen, bedeutet,
und n eine ganze Zahl von 2 bis 10 bedeutet, wobei die Chiralitätszentren in den Molekülen durch die verwendeten Edukte bestimmt werden und beide L oder beide D oder D,L bzw. L,D konfiguriert sein können.

Ein weiterer Gegenstand der Erfindung ist ein Verfahren zur Herstellung solcher asymmetrisch substituierter Diaminodicarbonsäurederivate durch gemischte Kolbe-Synthese, das dadurch gekennzeichnet ist, daß ein geschütztes Aminosäurederivat der Formel 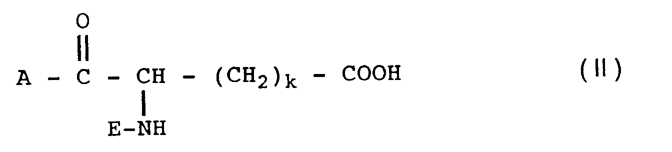
in der A und E die oben genannte Bedeutung haben und k eine ganze Zahl bedeutet,
mit einem Aminosäurederivat der Formel 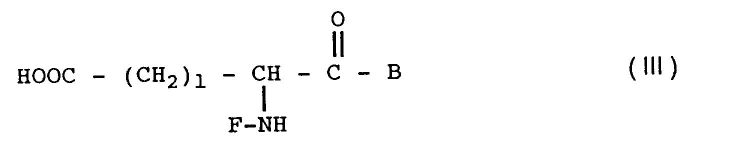
in der B und F die oben genannte Bedeutung haben und l eine ganze Zahl bedeutet, wobei k und l zusammen die Zahl n ergeben,
einer Elektrolyse an Platinnetzelektroden unterworfen wird.
In den Formeln I,II und III bedeuten A und B jeweils den Rest -OR, wobei R einen gegebenenfalls ein- oder mehrfach halogenierten geradkettigen oder verzweigten oder zyklischen Alkylrest mit 1-10 C-Atomen, beispielsweise einen gegebenenfalls halogenierten Methyl-, Ethyl-, i-Propyl-, n-Butyl-, i-Butyl-, t-Butylrest und dgl., einen Cyclohexyl-, Cyclopentyl- oder Cyclobutylrest oder Phenyl bedeutet.

Weiters kann R den Rest -CH₂-X bedeuten, wobei X die Reste 9-Fluorenyl-, Phenyl-, -OCH₃, -CH₂SO₂CH₃, -CH₂SO₂C₆H₅, -CCl₃, -CH₂-Y, mit Y Halogen, -p-Tosyl, einem gegebenenfalls ein- oder mehrfach durch Halogen, -NO₂ oder Alkoxy substituierten Phenyl- oder Phenacylrest, beispielsweise p-Bromphenacyl, p-Chlorphenacyl und dgl.,
Diphenylmethyl, Triphenylmethyl, 2-Pyridyl-, oder einem Rest -SiR₁R₂R₃, wobei die Reste R₁R₂R₃ jeweils unabhängig voneinander einen geradkettigen oder verzweigten Alkylrest mit 1-4 C-Atomen oder Phenyl bedeuten können, bedeutet.

Vorzugsweise bedeuten A und B einen Rest -OR, wobei R 9-Fluorenylmethyl-, einen substituierten oder unsubstituierten Phenyl-, Benzyl- oder Phenacylrest oder einen gegebenenfalls halogenierten geradkettigen oder verzweigten Alkylrest mit 1-4 C-Atomen bedeutet.

Die Reste E und F bedeuten jeweils einen gegebenenfalls halogenierten geradkettigen, verzweigten oder zyklischen Alkylrest mit 1-10 C-Atomen, beispielsweise einen Methyl-, Ethyl-, n-Propyl-, i-Propyl-, n-Butyl-, i-Butyl, t-Butyl, Pentyl-, Hexylrest und dgl., der gegebenenfalls ein- oder mehrfach halogeniert sein kann.

Ferner können die Reste E und F einen Rest 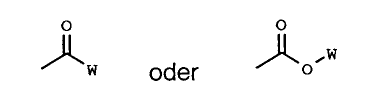
bedeuten, wobei W 9-Fluorenylmethyl- oder einen gegebenenfalls ein- oder mehrfach oder gemischt durch Halogen, NO₂, Alkoxy oder -CN substituierten Benzylrest bedeutet, beispielsweise einen Brombenzyl-, Dibrombenzyl-, Chlorbenzyl-, Dichlorbenzyl-, Nitrobenzyl-, Methoxybenzyl, Cyanobenzylrest und dgl.

Weiters können die Substituenten E bzw. F, bei Wegfall des Wasserstoffatoms, eines der angegebenen Ringsysteme bilden.

Vorzugsweise bedeuten die Reste E und F jeweils einen Rest 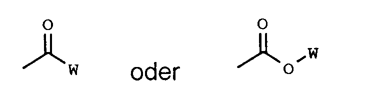
wobei W einen 9-Fluorenylmethylrest, einen gegebenenfalls substituierten Benzylrest oder einen geradkettigen oder verzweigten Alkylrest mit 1-4 C-Atomen bedeutet.

Wenn die Reste A und B unterschiedlich sind, können die Substituenten an der Aminofunktion der Dicarbonsäure gleich oder verschieden sein. Sind die Reste A und B gleich, so müssen die Substituenten an der Aminofunktion der Dicarbonsäure unterschiedlich sein.

Die Chiralitätszentren der Dicarbonsäuren werden durch die Wahl der verwendeten Edukte bestimmt. Sie können entweder beide D oder beide L oder D,L bzw. L,D, beispielsweise N'-E-N''-F-2,7-D,L-2,7-diaminosuberinsäuremono-A-ester-mono-B-ester bei Verwendung von N-E-D-glutaminsäure-A-ester und N-F-L-glutaminsäure-B-ester, konfiguriert sein.

Die erfindungsgemäßen asymmetrisch substituierten Dicarbonsäurederivate können durch gemischte Kolbe-Synthese hergestellt werden.
Dabei werden entsprechend geschützte Aminosäurederivate einer Elektrolyse an Platinnetzelektroden unterzogen.
Die Ausgangsverbindungen sind literaturbekannt oder können durch dem Fachmann geläufige Methoden hergestellt werden.

Die Aminosäurederivate werden dabei in einem unter Reaktionsbedingungen inerten Lösungsmittel gelöst. Als Lösungsmittel kommen beispielsweise niedere aliphatische Alkohole, beispielsweise Methanol, Ethanol, Propanol oder i-Propanol oder ein heterozyklisches Lösungsmittel wie beispielsweise Pyridin, oder Dimethylformamid, Azetonitril, Nitromethan oder Mischungen solcher Lösungsmittel in Frage.

In der Elektrolysezelle wird der Lösung eine Base zugefügt, beispielsweise Alkalimetall in alkoholischer Lösung, etwa Natriummethoxid in Methanol, oder Kaliumethoxid in Ethanol.
Anschließend wird an Platinnetzelektroden unter Kühlen elektrolysiert, wobei die Temperatur vorzugsweise auf 18-25°C gehalten wird. Die Stromstärke bei der Elektrolyse beträgt etwa 5-15 A bei 60-120 V angelegter Spannung und ist im übrigen abhängig von der Geometrie der verwendeten Elektrode.
Der Elektrolysevorgang wird beendet, sobald kein Edukt in der Elektrolyselösung mehr festgestellt werden kann.
Die Elektrolyselösung wird anschließend gegebenenfalls unter niedrigem Druck eingeengt, der Rückstand in einem geeigneten Lösungsmittel, beispielsweise Ethylacetat, aufgenommen und diese Lösung nacheinander mit verdünnter Säure, beispielsweise verdünnter Salzsäure, einer gesättigten Salzlösung, beispielsweise einer gesättigten Natriumhydrogencarbonatlösung, und gesättigter Natrium-chloridlösung gewaschen.
Die Lösung wird anschließend mit einem geeigneten Trocknungsmittel, beispielsweise Natriumsulfat oder Magnesiumsulfat, getrocknet, filtriert und erneut, gegebenenfalls unter niedrigem Druck, eingeengt.

Der Rückstand wird chromatographisch gereinigt, beispielsweise über Silicagel, wobei die symmetrisch substituierten Nebenprodukte abgetrennt werden können. Die Reaktion verläuft in guter Ausbeute von 10-15% d. Th. zum gewünschten asymmetrisch substituierten Endprodukt.

### Beispiel 1:

29,41 g (96 mmol) N-t-Butyloxycarbonylglutaminsäure-α-t-butylester und 35,63 g (96 mmol) N-Benzyloxycarbonylglutaminsäure-α-benzylester wurden in 240 ml MeOH und 80 ml Pyridin durch Umschwenken gelöst. Die Reaktionslösung wurde in die Elektrolysezelle mit zylinderförmig angeordneten Platinnetzelektroden transferiert. Es wurde mit MeOH nachgespült und die Elektrolysezelle soweit mit MeOH aufgefüllt, daß beide Elektroden vollständig eintauchten.
Nun wurden 0,8 ml NaOCH₃ (30% in MeOH) zugegeben, und die Elektrolysezelle gut gekühlt. Wenn die Reaktionslösung auf 15°C gekühlt ist, wird das Netzgerät eingeschaltet. Die Reaktionstemperatur wurde durch Temperaturregelung bzw. durch Regelung der Stromstärke bzw. -spannung (5-15 A, 60-120 V) zwischen +18°C und +24°C gehalten.
Der Reaktionsverlauf wurde mittels DC kontrolliert.
Nach vollständiger Reaktion wurde die Reaktionslösung bei 40 °C einrotiert.

Der Rückstand der Kolbe-Synthese wurde in 500 ml Ethylacetat gelöst, zuerst mit verdünnter HCl-Lösung (25 ml HCl conc. mit H₂O auf 250 ml aufgefüllt), dann mit 250 ml NaHCO₃ ges. und zuletzt mit je 250 ml NaCl ges. bis zur Neutralität der wäßrigen Phase gewaschen.

Die organische Phase wurde mit Na₂SO₄ getrocknet, abfiltriert, und eingedampft. Eindampfrückstand: 58,17 g.

Der Eindampfrückstand wurde über Kieselgel filtriert und anschließend mittels HPLC aufgetrennt.

Ausbeute: 4,5 g des reinen N'-Benzyloxycarbonyl-N''-t-Butyloxycarbonyl-2,7-diaminosuberinsäuremonobenzylester-mono-t-butylester (10% d.Th.), Fp. 51-56 C, [α]_{D}= -21,5°
Auf analoge Weise wurden folgende Verbindungen hergestellt:

| Nr. | A | B | E | F | n |
|---|---|---|---|---|---|
| 2 | O-Benzyl | O-Methyl | t-Butyloxycarbonyl | t-Butyloxycarbonyl | 4 |
| 3 | O-Benzyl | O-Benzyl | t-Butyloxycarbonyl | Benzyloxycarbonyl | 4 |
| 4 | O-Benzyl | O-2-Tosylethyl | t-Butyloxycarbonyl | Benzyloxycarbonyl | 4 |
| 5 | O-Benzyl | O-2-Tosylethyl | t-Butyloxycarbonyl | t-Butyloxycarbonyl | 4 |
| 6 | O-Benzyl | O-2-Tosylethyl | t-Butyloxycarbonyl | Benzyloxycarbonyl | 3 |
| 7 | O-Benzyl | O-2-Tosylethyl | t-Butyloxycarbonyl | Benzyloxycarbonyl | 2 |
| 8 | O-Benzyl | O-Methyl | t-Butyloxycarbonyl | Benzyloxycarbonyl | 3 |
| 9 | O-Benzyl | O-Phenacyl | t-Butyloxycarbonyl | Benzyloxycarbonyl | 3 |
| 10 | O-Benzyl | O-2-Trimethylsilethyl | t-Butyloxycarbonyl | Benzyloxycarbonyl | 3 |
| 11 | O-Benzyl | O-Benzyl | t-Butyloxycarbonyl | Benzyloxycarbonyl | 3 |
| 12 | O-Benzyl | O-2,2,2-Trichlorethyl | t-Butyloxycarbonyl | Benzyloxycarbonyl | 2 |
| 13 | O-Benzyl | O-Benzyl | t-Butyloxycarbonyl | Benzyloxycarbonyl | 2 |
| 14 | O-Benzyl | O-2-Tosylethyl | t-Butyloxycarbonyl | Benzyloxycarbonyl | 3 |

In den Beispielen 5 und 14 wurden die entsprechenden D- und L- Aminosäurederivate gemischt eingesetzt.

Chemische Daten der oben angeführten Verbindungen, wobei die verwendeten Abkürzungen folgende Bedeutung haben:

| Abkürzung | Bedeutung |
|---|---|
| OBn | O-Benzyl |
| OMe | O-Methyl |
| OEtTos | O-Ethyltosyl |
| OtBu | O-tert-Butyl |
| Boc | t-Butyloxycarbonyl |
| Z | Benzyloxycarbonyl |
| SUB | n=4 |
| PIM | n=3 |
| ADI | n=2 |

### Beispiel 1: Boc-Z-SUB-OtBU-OBn

¹³C(CDCl₃, 100 MHz): 24.80(CH₂), 24.82(CH₂), 28.02(O-t-Bu-CH₃), 28,34(Boc-CH₃), 32.52(CH₂), 32.72(CH₂), 53.82(CH), 67.00 und 67.12(Benzyl-**C**H₂), 79.62((CH₃)₃**C** von Boc), 81.57((CH₃)₃**C** von OtBu), 128.09-128.63(Aromaten-C), 135.36, 136.30, 155.34 und 155.87(Carbamat-CO), 171.86 und 172.22(CO).
Fp.51-56°C
[α]_{D}= -21.5

### Beispiel 2: Di-Boc-SUB-OBn-OMe

¹³C(CDCl₃, 100MHz): 24.82(2CH₂), 28.31 (2(**C**H₃)₃C), 32.49(CH₂), 32.54(CH₂), 52.18(O**C**H₃), 53.38(br s, 2CH), 66.99(Benzyl-**C**H₂), 128.31, 128.42, 128.59, 135.46,'und 155.32(2 Carbamat-CO), 172.57 und 173.20(Ester-CO).
Fp.55-59°C
[α]_{D}= -24.9(1% in DMF)

### Beispiel 3: Boc-Z-SUB-Di-OBn

¹³C(CDCl₃, 100MHz): 24.68(CH₂), 24.81(CH₂), 28.32(2 (**C**H₃)₃C), 32.47(2CH₂), 53.37(CH), 53.83(CH), 66.99(Benzyl-**C**H₂), 67.13(Benzyl-**C**H₂), 79.90((CH₃)₃**C**), 128.10-128.63(Aromaten-C), 135.33 135.46, 136.28, 155.30 und 155.83(Carbamat-CO), 172.17 und 172.56(Ester-CO)
Fp. 65-67°C
[α]_{D}= -1.4(5% in CHCl₃)

### Beispiel 4: Boc-Z-SUB-OBn-OEtTos

¹³C(CDCl₃, 100MHz): 21.65(Tolyl-CH₃), 24.65(CH₂), 24.81(CH₂), 28.32((**C**H₃)₃C), 32.06(CH₂), 32.38(CH₂), 53.12(CH), 53.80(CH), 54.94(O**C**H₂CH₂SO₂C₇H₇), 58.27(OCH₂**C**H₂SO₂C₇H₇), 67.01 (Benzyl-**C**H₂), 67.17(Benzyl-**C**H₂), 80.05((CH₃)₃**C**), 128.12-128.65(Aromaten-C), 130.08, 135.32, 136.27, 145.23, 155.26 und 155.90(Carbamat-CO), 172.15(2 Ester-CO)
Öl
[α]_{D}= +3.45 (5% in CHCl₃)

### Beispiel 5: Di-Boc-D,L-SUB-OBn-OEtTos

¹³C(CDCl₃, 100MHz): 21.63(Tolyl-CH₃), 24.79(CH₂), 24.84(CH₂), 28.31(2(**C**H₃)₃C), 32.13(CH₂), 32.52(CH₂), 53.23(br s, 2CH), 54.99(O**C**H₂CH₂SO₂C₇H₇), 58.28(OCH₂**C**H₂SO₂C₇H₇), 67.01 (Benzyl-**C**H₂), 79.98(2(CH₃)₃**C**), 128.13-128.61(Aromaten-C), 135.45, 136.35, 145.21, 155.30 und 155.83(Carbamat-CO), 172.11(2 Ester-CO)

### Beispiel 6: Boc-Z-PIM-OBn-OEtTos

¹³C(CDCl₃, 100MHz): 21.10(CH₂), 21.60(Tolyl-CH₃), 28.31 ((**C**H₃)₃C), 31.62(CH₂), 31.90(CH₂), 52.85(CH), 53.55(CH), 54.93(O**C**H₂CH₂SO₂C₇H₇), 58.32(OCH₂**C**H₂SO₂C₇H₇), 67.05 und 67.22(Benzyl-**C**H₂), 80.08((CH₃)₃**C**), 128.11-128.66(Aromaten-C), 130.05, 135.31, 136.25, 145.21, 155.45 und 155.83(Carbamat-CO), 171.97 und 172.08(Ester-CO)

### Beispiel 7: Boc-Z-ADI-OBn-OEtTos

¹³C(CDCl₃, 100MHz): 21.60(Tolyl-CH₃), 28.17((**C**H₃)₃C), 28.29(CH₂), 52.79(CH), 53.61(CH), 54.87(O**C**H₂CH₂SO₂C₇H₇), 58.29(OCH₂**C**H₂SO₂C₇H₇), 67.04 und 67.31 (Benzyl-**C**H₂), 80.18((CH₃)₃**C**), 128.05-128.67(Aromaten-C), 130.09, 135.24, 136.20, 136.26, 145.27 und 156.00(2 Carbamat-CO), 171.59 und 171.75(Ester-CO)

### Beispiel 8: Boc-Z-PIM-OBn-OMe

¹³C(CDCl₃, 100MHz): 20.82(CH₂), 21.16(CH₂), 28.31((**C**H₃)₃C), 31.97(CH₂), 32.17(CH₂), 52.23(OMe), 52.93(CH), 53.61(CH), 67.07(Benzyl-**C**H₂), 80.01((CH₃)₃**C**), 128.17-128.64(Aromaten-C), 135.29, 136.23, 155.55 und 156.06(Carbamat-CO), 172.12 und 173.06(Ester-CO)

### Beispiel 9: Boc-Z-PIM-OBn-OCH₂COC₆H₅

¹³C(CDCl₃, 100MHz): 20.82(2CH₂), 28.33((**C**H₃)₃C), 31.86(CH₂), 32.16(CH₂), 53.08(CH), 53.69(CH), 66.35(O**C**H₂COC₆H₅), 66.97(Benzyl-**C**H₂), 67.13(Benzyl-**C**H₂), 80.05((CH₃)₃**C**), 127.77-128.89(Aromaten-C), 133.99, 135.43, 136.36, 155.51 und 156.17(Carbamat-CO), 172.16(2 Ester-CO), 191.61(OCH₂**C**OC₆H₅)

### Beispiel 10: Boc-Z-PIM-OBn-OCH₂-CH₂Si(CH₃)₃

¹³C(d₆-DMSO, 100MHz): -1.54((CH₃)₃Si), 17.42(OCH₂**C**H₂Si(CH₃)₃), 21.22(CH₂), 22.20(CH₂), 28.32((**C**H₃)₃C), 31.91(CH₂), 32.34(CH₂), 53.01(CH), 53.73(CH), 63.74(O**C**H₂CH₂Si(CH₃)₃), 67.05(Benzyl-**C**H₂), 67.17(Benzyl-**C**H₂), 79.89((CH₃)₃**C**), 128.14, 128.26, 128.50, 128.64, 135.32, 136.25, 155.58 und 156.06(Carbamat-CO), 172.17 und 172.68(2 Ester-CO)

### Beispiel 11: Boc-Z-PIM-Di-OBn

¹³C(CDCl₃, 100MHz): 21.14(CH₂), 28.30((**C**H₃)₃C), 31.92(CH₂), 32.16(CH₂), 53.07(CH), 53.64(CH),, 67.06(2 benzyl-**C**H₂), 67.18(benzyl-**C**H₂), 79.98((CH₃)₃**C**), 128.16, 128.29, 128.44, 128.50, 128.61, 128.63, 135.30, 135.40, 136.24, 155.53 und 156.04(carbamat-CO), 172.09 und 172.41(2 ester-CO).

### Beispiel 12: Boc-Z-ADI-OBn-OCH₂CCl₃

¹³C(CDCl₃, 100MHz): 28.60((**C**H₃)₃C), 29.25(CH₂), 30.00(CH₂), 53.34(CH), 53.78(CH), 74.67(O**C**H₂CCl₃), 67.47(benzyl-**C**H₂), 67.72(benzyl-**C**H₂), 74.67(O**C**H₂CCl₃), 80.69((CH₃)₃**C**), 94.79(OCH₂**C**Cl₃), 128.42, 128.54, 128.74, 128.87, 128.96, 129.02, 135.43, 136.46, 155.53 und 156.17(carbamat-CO), 171.09 und 171.96(2 CO).

### Beispiel 13: Boc-Z-ADI-Di-OBn

¹³C(CDCL₃, 100MHz): 21.22(CH₂), 22.20(CH₂), 28.32((**C**H₃)₃C), 31.91(CH₂), 32.34(CH₂), 53.01(CH), 53.73(CH), 63.74(O**C**H₂CH₂Si(CH₃)₃), 67.05(benzyl-**C**H₂), 67.17(benzyl-**C**H₂), 79.89((CH₃)₃**C**), 128.14, 128.26, 128.50, 128.64, 135.32, 136.25, 155.58 und 156.06(carbamat-CO), 172.17 und 172.68(2 ester-CO).

### Beispiel 14: Boc-Z-D,L-PIM-OBn-OEtTos

¹³C(CDCl₃, 100MHz): 20.98(CH₂), 21.58(tolyl-CH₃), 28.29((**C**H₃)₃C), 31.77(CH₂), 31.93(CH₂), 52.98(CH), 53.73(CH), 54.94(O**C**H₂CH₂SO₂C₇H₇), 58.25(OCH₂**C**H₂SO₂C₇H₇), 67.00 und 67.15(benzyl-**C**H₂), 80.10((CH₃)₃**C**), 128.09-128.64(aromaten-C), 130.05, 135.31, 136.32, 145.19, 155.28 und 156.02(carbamat-CO), 171.87 und 171.94 (ester-CO).

## Patentansprüche

1. Asymmetrisch substituierte Diaminodicarbonsäurederivate der Formel 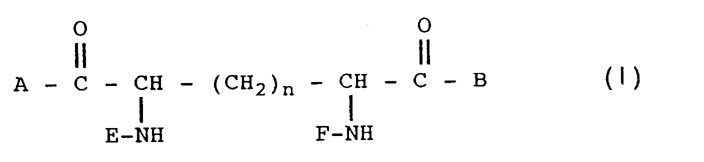 in der
A und B unabhängig voneinander jeweils einen Rest -OR, wobei R einen gegebenenfalls ein- oder mehrfach halogenierten geradkettigen, verzweigten oder zyklischen Alkylrest mit 1-10 C-Atomen, Phenyl oder den Rest -CH₂-X, wobei X die Reste 9-Fluorenyl-, Phenyl-, -OCH₃, -CH₂SO₂CH₃, - CH₂SO₂C₆H₅, -CCl₃, -CH₂-Y, mit Y Halogen, -p-Tosyl, einem gegebenenfalls ein- oder mehrfach durch Halogen, -NO₂ oder Alkoxy substituierten Phenyl- oder Phenacylrest, Diphenylmethyl, Triphenylmethyl, 2-Pyridyl-, oder einem Rest SiR₁R₂R₃, wobei die Reste R₁R₂R₃ jeweils unabhängig voneinander einen geradkettigen oder verzweigten Alkylrest mit 1-4 C-Atomen oder Phenyl bedeuten können, bedeutet,
E, F jeweils einen gegebenenfalls halogenierten geradkettigen, verzweigten oder cyklischen Alkylrest mit 1-10 C-Atomen oder einen Rest 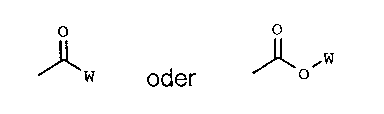 bedeuten, wobei W 9-Fluorenylmethyl- oder einen gegebenenfalls ein- oder mehrfach oder gemischt durch Halogen, -NO₂, Alkoxy oder -CN substituierten Benzylrest bedeuten kann, oder die Substituenten E bzw. F, bei Wegfall des Wasserstoffatoms, gemeinsam mit dem Stickstoffatom eines der folgenden Ringsysteme bilden 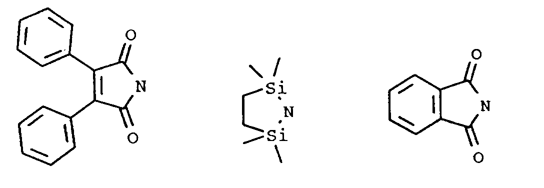 wobei, wenn die Reste A und B unterschiedlich sind, E und F unterschiedlich oder gleich sein können, hingegen wenn die Reste A und B gleich sind, E und F unterschiedlich sein müssen, bedeutet,
und n eine ganze Zahl von 2 bis 10 bedeutet, wobei die Chiralitätszentren in den Molekülen durch die verwendeten Edukte bestimmt werden und beide L oder beide D oder D,L bzw. L,D konfiguriert sein können.

2. Asymmetrisch substituierte Diaminodicarbonsäurederivate der Formel I nach Anspruch 1, wonach A und B einen Rest -OR bedeuten, wobei R 9-Fluorenylmethyl-, einen substituierten oder unsubstituierten Phenyl-, Benzyl- oder Phenacylrest, einen 2-(2-Pyridyl)-ethylrest, einen 2-p-Tosylethylrest oder einen gegebenenfalls halogenierten geradkettigen oder verzweigten Alkylrest mit 1-4 C-Atomen bedeutet,
E und F jeweils einen Rest 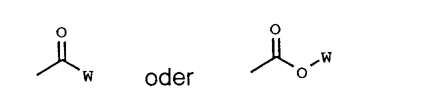 bedeuten, wobei W einen 9-Fluorenylmethylrest, einen gegebenenfalls substituierten Benzylrest oder einen geradkettigen oder verzweigten Alkylrest mit 1-4 C-Atomen bedeutet und n eine ganze Zahl von 2 bis 10 bedeutet.

3. Verfahren zur Herstellung solcher asymmetrisch substituierten Diaminodicarbonsäurederivate durch gemischte Kolbesynthese, das dadurch gekennzeichnet ist, daß ein geschütztes Aminosäurederivat der Formel 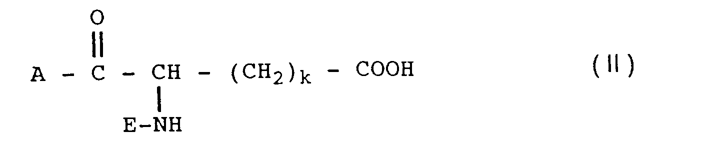 in der A und E die oben genannte Bedeutung haben und k eine ganze Zahl bedeutet,
mit einem Aminosäurederivat der Formel 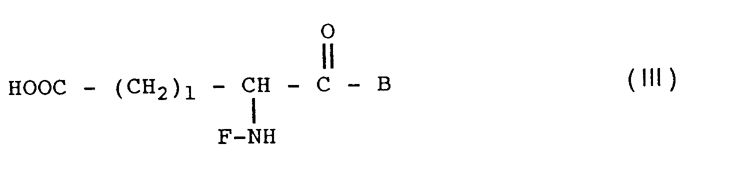 in der B und F die oben genannte Bedeutung haben und l eine ganze Zahl bedeutet, wobei k und l zusammen die Zahl n ergeben,
einer Elektrolyse an Platinnetzelektroden unterworfen wird.

4. Ein Verfahren nach Anspruch 3, dadurch gekennzeichnet, daß die Temperatur bei der Elektrolyse durch Kühlen zwischen 18 - 25°C gehalten wird.
